# EUROPEAN PATENT APPLICATION

(11) **EP 4 098 261 A1**
(43) Date of publication of application: **07.12.2022**
(21) Application number: 21768185.7
(22) Date of filing: 10.03.2021
(51) Int. Cl.: A61K 31/506, A61K 31/4985, A61K 31/495, A61K 31/192, A61P 1/16

(54) **PHARMACEUTICAL COMPOSITION FOR PREVENTION OR TREATMENT OF NONALCOHOLIC STEATOHEPATITIS**

(30) Priority: 11.03.2020 KR 20200030423
(71) Applicant: Dong-A ST Co., Ltd., Dongdaemun-gu Seoul 02587 (KR)
(72) Inventor: KIM, Mi-Kyung, Suwon-Si Gyeonggi-do 16505 (KR); PARK, Hansu, Suwon-Si Gyeonggi-do 16693 (KR); LEE, Seung Ho, Yongin-si Gyeonggi-do 17095 (KR)
(74) Representative: Gille Hrabal Partnerschaftsgesellschaft mbB Patentanwälte
(86) International application number: PCT/KR2021/002989
(87) International publication number: WO 2021/182876

(57) **Abstract**

The present invention relates to a pharmaceutical composition for preventing or treating nonalcoholic fatty liver disease. The pharmaceutical composition according to the present invention exhibits excellent effects of improving lipid metabolism, reducing fat accumulation in liver tissues and inhibiting histological damage caused by inflammation and fibrosis of liver tissues, and thus can be effectively used as a use of preventing or treating nonalcoholic fatty liver disease.

## Description

### Technical Field

The present invention relates to a pharmaceutical composition for preventing or treating nonalcoholic fatty liver disease, including a G protein coupled receptor 119 (GPR119) agonist as an active ingredient and at least one drug of another mechanism.

### Background

Fatty liver is a pathological condition in which triglycerides are excessively accumulated in hepatocytes, and medically defined as a condition in which triglycerides account for 5% or more of a liver weight. Fatty liver is classified into alcoholic and nonalcoholic fatty livers depending on whether fatty liver is caused by excessive alcohol intake or not. Nonalcoholic fatty liver disease (NAFLD) is a group of diseases that encompass all aspects of the disease ranging from nonalcoholic fatty liver to steatohepatitis and cirrhosis. Simple nonalcoholic fatty liver is a disease in which only fat deposition in the liver is present, but without any findings about hepatocyte damage and fibrosis, and only fat deposition in liver tissues is increased due to insulin resistance, etc. Nonalcoholic fatty liver may progress into nonalcoholic steatohepatitis (NASH) accompanied by fibrosis due to hepatocyte damage caused by an inflammatory response according to oxidative stress or the like.

Unlike simple fatty liver, nonalcoholic steatohepatitis may progress into cirrhosis and liver cancer causing irreversible liver damage, thus increasing mortality associated with liver disease and overall mortality (Hepatology, 2012(55):2005-2023). For this reason, as of 2013, nonalcoholic steatohepatitis was a second most common cause of liver transplantation after hepatitis C according to US statistical data. Since 2020, however, nonalcoholic steatohepatitis has been expected to become a first causative disease of liver transplantation rather than hepatitis C, and thus there is an urgent need for developing a drug for preventing and treating nonalcoholic steatohepatitis (Clinical Gastroenterology and Hepatology, 2019(17):748-755).

### Detailed Description of the Invention

### Technical Problem

One object of the present invention is to provide a pharmaceutical composition for preventing or treating nonalcoholic steatohepatitis, which includes a compound represented by chemical Formula 1, pharmaceutically acceptable salts thereof, optical isomers thereof, hydrates thereof, solvates thereof, or mixtures thereof; and at least one selected from a PPAR agonist, a DPPIV inhibitor, an ACC inhibitor, a FXR agonist and a CCR2/5 dual antagonist.

### Technical Solution

A pharmaceutical composition for preventing or treating nonalcoholic steatohepatitis according to one embodiment of the present invention may include a compound represented by chemical Formula 1 below, pharmaceutically acceptable salts thereof, optical isomers thereof, hydrates thereof, solvates thereof, or mixtures thereof; and at least one selected from a PPAR agonist, a DPPIV inhibitor, an ACC inhibitor, a FXR agonist and a CCR2/5 dual antagonist.

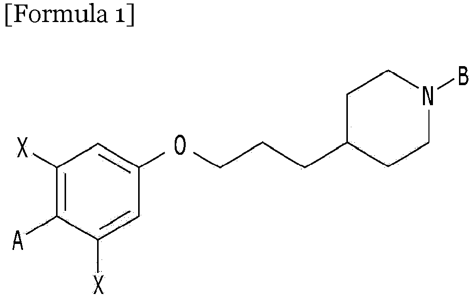

In above Formula 1, A may be an oxadiazole group, a dihydrooxazole group, a thiazole group or a thiadiazole group. Above A may be unsubstituted or substituted with at least one substituent selected from the group consisting of a halogen atom, a C1-C6 straight or branched chain alkyl group and a C1-C6 straight or branched chain hydroxyalkyl group. The alkyl group or the hydroxyalkyl group may be each independently unsubstituted or substituted with a halogen atom or a C1-C6 alkoxy group.

B may be a pyridine group, a pyrimidine group, a pyrazine group or an oxadiazole group. Above B may be unsubstituted or substituted with at least one substituent selected from the group consisting of a halogen atom, a C1-C6 straight or branched chain alkyl group, a C1-C6 straight or branched chain hydroxyalkyl group, a C1-C6 alkoxy group and an oxadiazole group. The C1-C6 straight or branched chain alkyl group, the C1-C6 straight or branched chain hydroxyalkyl group, the C1-C6 alkoxy group or the oxadiazole group may be each independently unsubstituted or substituted with halogen, a C1-C6 alkyl group or a C1-C6 alkoxy group.

X may be F, Cl, Br or I.

Above A may be

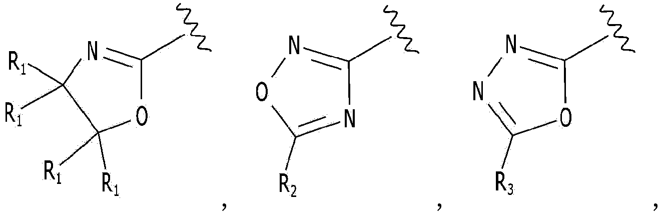

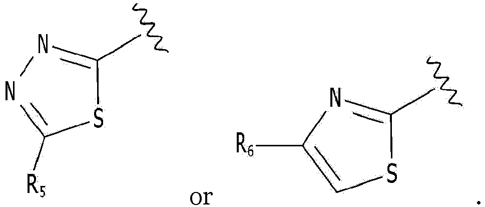

R₁ to R₃, R₅, and R₆ may be each independently at least one substituent selected from the group consisting of a hydrogen atom, a halogen atom, a C1-C6 straight or branched chain alkyl group and a C1-C6 straight or branched chain hydroxyalkyl group. The alkyl group or the hydroxyalkyl group may be each independently unsubstituted or substituted with a halogen atom or a C1-C6 alkoxy group.

Above B may be

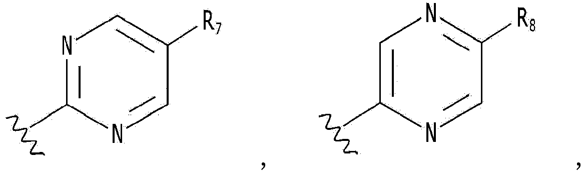

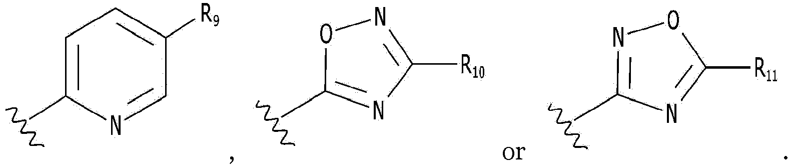

Above R₇ to R₁₁ may be each independently substituted with at least one substituent selected from the group consisting of a hydrogen atom, a halogen atom, a C1-C6 straight or branched chain alkyl group, a C1-C6 straight or branched chain hydroxyalkyl group, a C1-C6 alkoxy group and an oxadiazole group. The alkyl group, the hydroxyalkyl group, the alkoxy group or the oxadiazole group may be each independently unsubstituted or substituted with a halogen atom, a C1-C6 alkyl group or a C1-C6 alkoxy group.

X may be F.

In above Formula 1, above A may be an oxadiazole group substituted with a C1-C6 straight or branched chain alkyl group, above B may be a pyrimidine group substituted with a C1-C6 straight or branched chain alkyl group, and X may be F.

In the present embodiment, the term "halogen" may refer to fluorine, chlorine, bromine or iodine.

In one embodiment, the term "alkyl" may refer to a straight or branched chain hydrocarbon residue unless otherwise specified. An example of the C1-C6 alkyl may include methyl, ethyl, propyl, isopropyl, butyl, isobutyl, pentyl, hexyl, etc.

In one embodiment, the term "alkoxy" may include an alkyl-oxygen radical having alkyl as defined above unless otherwise specified. An example of the C1-C6 alkoxy may include methoxy, ethoxy, propoxy, butoxy, pentoxy, etc.

In one embodiment, the term "heterocycle" or "heterocyclic" may refer to a 5 to 13-membered hetero-aromatic or non-aromatic compound including 1 to 3 heteroatoms selected from the group consisting of N, O, and S unless otherwise specified.

In one embodiment, the compound represented by above Formula 1 may be at least one compound specifically selected from the group consisting of the following compounds:
2-(4-(3-(1-(5-ethylpyrimidin-2-yl)piperidin-4-yl)propoxy)-2,6-difluorophenyl)-4,5-dihydrooxazole,
(R)-2-(4-(3-(1-(5-ethylpyrimidin-2-yl)piperidin-4-yl)propoxy)-2,6-difluorophenyl)-4-methyl-4,5-dihydrooxazole,
(S)-2-(4-(3-(1-(5-ethylpyrimidin-2-yl)piperidin-4-yl)propoxy)-2,6-difluorophenyl)-4-methyl-4,5-dihydrooxazole,
(S)-2-(4-(3-(1-(5-ethylpyrimidin-2-yl)piperidin-4-yl)propoxy)-2,6-difluorophenyl)-5-methyl-4,5-dihydrooxazole,
(R)-2-(4-(3-(1-(5-ethylpyrimidin-2-yl)piperidin-4-yl)propoxy)-2,6-difluorophenyl)-5-methyl-4,5-dihydrooxazole,
2-(4-(3-(1-(5-ethylpyrimidin-2-yl)piperidin-4-yl)propoxy)-2,6-difluorophenyl)-5,5-dimethyl-4,5-dihydrooxazole,
(R)-(2-(4-(3-(1-(5-ethylpyrimidin-2-yl)piperidin-4-yl)propoxy)-2,6-difluorophenyl)-4,5-dihydrooxazole-5-yl)methanol,
(S)-(2-(4-(3-(1-(5-ethylpyrimidin-2-yl)piperidin-4-yl)propoxy)-2,6-difluorophenyl)-4,5-dihydrooxazole-5-yl)methanol,
(R)-3-(2-(4-(3-(3,5-difluoro-4-(5-methyl-4,5-dihydrooxazole-2-yl)phenoxy)propyl)piperidin-1-yl)pyrimidin-5-yl)-5-isobutyl-1,2,4-oxadiazole,
(R)-5-(4-(3-(3,5-difluoro-4-(4-methyl-4,5-dihydrooxazol-2-yl)phenoxy)propyl)piperidin-1-yl)-3-isopropyl-1,2,4-oxadiazole,
(S)-5-(4-(3-(3,5-difluoro-4-(5-methyl-4,5-dihydrooxazol-2-yl)phenoxy)propyl)piperidin-1-yl)-3-isopropyl-1,2,4-oxadiazole,
5-(4-(3-(4-(5,5-dimethyl-4,5-dihydrooxazol-2-yl)-3,5-difluorophenoxy)propyl)piperidin-1-yl)-3-isopropyl-1,2,4-oxadiazole,
3-(4-(3-(1-(5-ethylpyrimidin-2-yl)piperidin-4-yl)propoxy)-2,6-difluorophenyl)-5-methyl-1,2,4-oxadiazole,
3-(4-(3-(1-(5-ethylpyrimidin-2-yl)piperidin-4-yl)propoxy)-2,6-difluorophenyl)-5-propyl-1,2,4-oxadiazole,
3-(4-(3-(1-(5-ethylpyrimidin-2-yl)piperidin-4-yl)propoxy)-2,6-difluorophenyl)-5-isopropyl-1,2,4-oxadiazole,
5-(tert-butyl)-3-(4-(3-(1-(5-ethylpyrimidin-2-yl)piperidin-4-yl)propoxy)-2,6-difluorophenyl)-1,2,4-oxadiazole,
(3-(4-(3-(1-(5-ethylpyrimidin-2-yl)piperidin-4-yl)propoxy)-2,6-difluorophenyl)-1,2,4-oxadiazol-5-yl)methanol,
2-(3-(4-(3-(1-(5-ethylpyrimidin-2-yl)piperidin-4-yl)propoxy)-2,6-difluorophenyl)-1,2,4-oxadiazol-5-yl)ethan-1-ol,
(S)-1-(3-(4-(3-(1-(5-ethylpyrimidin-2-yl)piperidin-4-yl)propoxy)-2,6-difluorophenyl)-1,2,4-oxadiazole-5-yl)propan-1-ol,
(R)-1-(3-(4-(3-(1-(5-ethylpyrimidin-2-yl)piperidin-4-yl)propoxy)-2,6-difluorophenyl)-1,2,4-oxadiazol-5-yl)propane-2-ol,
(S)-1-(3-(4-(3-(1-(5-ethylpyrimidin-2-yl)piperidin-4-yl)propoxy)-2,6-difluorophenyl)-1,2,4-oxadiazol-5-yl)propan-2-ol,
2-(3-(4-(3-(1-(5-ethylpyrimidin-2-yl)piperidin-4-yl)propoxy)-2,6-difluorophenyl)-1,2,4-oxadiazol-5-yl)-2-methylpropan-1-ol,
3-(2,6-difluoro-4-(3-(1-(5-propylpyrimidin-2-yl)piperidin-4-yl)propoxy)phenyl)-5-isopropyl-1,2,4-oxadiazole,
3-(2,6-difluoro-4-(3-(1-(5-pentylpyrimidin-2-yl)piperidin-4-yl)propoxy)phenyl)-5-isopropyl-1,2,4-oxadiazole,
3-(2,6-difluoro-4-(3-(1-(5-(trifluoromethyl)pyrimidin-2-yl)piperidin-4-yl)propoxy)phenyl)-5-isopropyl-1,2,4-oxadiazole,
3-(2,6-difluoro-4-(3-(1-(5-methoxypyrimidin-2-yl)piperidin-4-yl)propoxy)phenyl)-5-isopropyl-1,2,4-oxadiazole,
3-(2,6-difluoro-4-(3-(1-(5-isopropoxypyrimidin-2-yl)piperidin-4-yl)propoxy)phenyl)-5-isopropyl-1,2,4-oxadiazole,
3-(4-(3-(1-(5-chloropyrimidin-2-yl)piperidin-4-yl)propoxy)-2,6-difluorophenyl)-5-isopropyl-1,2,4-oxadiazole,
3-(4-(3-(1-(5-bromopyrimidin-2-yl)piperidin-4-yl)propoxy)-2,6-difluorophenyl)-5-isopropyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-methyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-ethyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-isopropyl-1,2,4-oxadiazole,
5-(sec-butyl)-3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-(methoxymethyl)-1,2,4-oxadiazole,
(S)-1-(3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazol-5-yl)propan-1-ol,
2-(3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazol-5-yl)-2-methylpropan-1-ol,
3-(4-(3-(1-(5-chloropyrazin-2-yl)piperidin-4-yl)propoxy)-2,6-difluorophenyl)-5-isopropyl-i,2,4-oxadiazole,
3-(2,6-difluoro-4-(3-(1-(5-(trifluoromethyl)pyridin-2-yl)piperidin-4-yl)propoxy)phenyl)-5-isopropyl-1,2,4-oxadiazole,
3-(2,6-difluoro-4-(3-(1-(3-isopropyl-1,2,4-oxadiazol-5-yl)piperidin-4-yl)propoxy)phenyl)-5-methyl-1,2,4-oxadiazole,
3-(2,6-difluoro-4-(3-(1-(3-isopropyl-1,2,4-oxadiazol-5-yl)piperidin-4-yl)propoxy)phenyl)-5-isopropyl-1,2,4-oxadiazole,
(3-(2,6-difluoro-4-(3-(1-(3-isopropyl-1,2,4-oxadiazol-5-yl)piperidin-4-yl)propoxy)phenyl)-1,2,4-oxadiazol-5-yl)methanol,
2-(4-(3-(1-(5-ethylpyrimidin-2-yl)piperidin-4-yl)propoxy)-2,6-difluorophenyl)-5-methyl-1,3,4-oxadiazole,
2-ethyl-5-(4-(3-(1-(5-ethylpyrimidin-2-yl)piperidin-4-yl)propoxy)-2,6-difluorophenyl)-1,3,4-oxadiazole,
2-(4-(3-(1-(5-ethylpyrimidin-2-yl)piperidin-4-yl)propoxy)-2,6-difluorophenyl)-5-isopropyl-1,3,4-oxadiazole,
5-(4-(3-(1-(5-ethylpyrimidin-2-yl)piperidin-4-yl)propoxy)-2,6-difluorophenyl)-N-isopropyl-1,3,4-oxadiazol-2-amine,
2-(2,6-difluoro-4-(3-(1-(5-(trifluoromethyl)pyrimidin-2-yl)piperidin-4-yl)propoxy)phenyl)-5-methyl-1,3,4-oxadiazole,
2-(2,6-difluoro-4-(3-(1-(5-(trifluoromethyl)pyrimidin-2-yl)piperidin-4-yl)propoxy)phenyl)-5-ethyl-1,3,4-oxadiazole,
2-(2,6-difluoro-4-(3-(1-(5-(trifluoromethyl)pyrimidin-2-yl)piperidin-4-yl)propoxy)phenyl)-5-isopropyl-1,3,4-oxadiazole,
2-(4-(3-(1-(5-chloropyrazin-2-yl)piperidin-4-yl)propoxy)-2,6-difluorophenyl)-5-methyl-1,3,4-oxadiazole,
2-(4-(3-(1-(5-chloropyrazin-2-yl)piperidin-4-yl)propoxy)-2,6-difluorophenyl)-5-ethyl-1,3,4-oxadiazole,
2-(4-(3-(1-(5-chloropyrazin-2-yl)piperidin-4-yl)propoxy)-2,6-difluorophenyl)-5-isopropyl-1,3,4-oxadiazole,
5-(4-(3-(3,5-difluoro-4-(5-methyl-1,3,4-oxadiazol-2-yl)phenoxy)propyl)piperidin-1-yl)-3-propyl-1,2,4-oxadiazole,
5-(4-(3-(3,5-difluoro-4-(5-ethyl-1,3,4-oxadiazol-2-yl)phenoxy)propyl)piperidin-1-yl)-3-propyl-1,2,4-oxadiazole,
5-(4-(3-(3,5-difluoro-4-(5-isopropyl-1,3,4-oxadiazol-2-yl)phenoxy)propyl)piperidin-1-yl)-3-propyl-1,2,4-oxadiazole,
5-(4-(3-(3,5-difluoro-4-(5-methyl-1,3,4-oxadiazol-2-yl)phenoxy)propyl)piperidin-1-yl)-3-isopropyl-1,2,4-oxadiazole,
5-(4-(3-(4-(5-ethyl-1,3,4-oxadiazol-2-yl)-3,5-difluorophenoxy)propyl)piperidin-1-yl)-3-isopropyl-1,2,4-oxadiazole,
5-(4-(3-(3,5-difluoro-4-(5-isopropyl-1,3,4-oxadiazol-2-yl)phenoxy)propyl)piperidin-1-yl)-3-isopropyl-1,2,4-oxadiazole,
5-(4-(3-(3,5-difluoro-4-(5-methyl-1,3,4-oxadiazol-2-yl)phenoxy)propyl)piperidin-1-yl)-3-(2,2,2-trifluoroethyl)-1,2,4-oxadiazole,
3-(4-(3-(3,5-difluoro-4-(5-isopropyl-1,3,4-oxadiazol-2-yl)phenoxy)propyl)piperidin-1-yl)-5-isopropyl-1,2,4-oxadiazole,
2-(4-(3-(1-(5-ethylpyrimidin-2-yl)piperidin-4-yl)propoxy)-2,6-difluorophenyl)-5-isopropyl-1,3,4-thiadiazole,
2-(2,6-difluoro-4-(3-(1-(5-propylpyrimidin-2-yl)piperidin-4-yl)propoxy)phenyl)-5-isopropyl-1,3,4-thiadiazole,
2-(2,6-difluoro-4-(3-(1-(5-pentylpyrimidin-2-yl)piperidin-4-yl)propoxy)phenyl)-5-isopropyl-1,3,4-thiadiazole,
2-(2,6-difluoro-4-(3-(1-(5-fluoropyrimidin-2-yl)piperidin-4-yl)propoxy)phenyl)-5-isopropyl-1,3,4 - thiadiazole,
2-(2,6-difluoro-4-(3-(1-(5-(trifluoromethyl)pyrimidin-2-yl)piperidin-4-yl)propoxy)phenyl)-5-isopropyl-1,3,4-thiadiazole,
2-(2,6-difluoro-4-(3-(1-(5-(trifluoromethyl)pyridin-2-yl)piperidin-4-yl)propoxy)phenyl)-5-isopropyl-1,3,4-thiadiazole,and
4-ethyl-2-(4-(3-(1-(5-ethylpyrimidin-2-yl)piperidin-4-yl)propoxy)-2,6-difluorophenyl)thiazole.

In one embodiment, the compound represented by above Formula 1 may be 3-(4-(3-(1-(5-ethylpyrimidin-2-yl)piperidin-4-yl)propoxy)-2,6-difluorophenyl)-5-isopropyl-1,2,4-oxadiazole.

The pharmaceutical composition of one embodiment may include a compound represented by above Formula 1; and any one selected from the PPAR agonist, the DPPIV inhibitor, the ACC inhibitor, the FXR agonist and the CCR2/5 dual antagonist.

The PPAR agonist may be at least one of pioglitazone, lobeglitazone, elafibranor, lanifibranor and saroglitazar. The DPPIV inhibitor may be at least one of sitagliptin, vildagliptin, saxagliptin, linagliptin, gemigliptin, anagliptin, teneligliptin, alogliptin, trelagliptin, omarigliptin, evogliptin, gosogliptin and dutogliptin. The ACC inhibitor may be firsocostat. The FXR agonist may be at least one of obeticholic acid, tropifexor and GW4064 (3-(2,6-dichlorophenyl)-4-(3'-carboxy-2-chlorostilben-4-yl)oxymethyl-5-isopropylisoxazole). The CCR 2/5 dual antagonist may be cenicriviroc.

For example, the pharmaceutical composition may include a compound represented by above Formula 1 and the PPAR agonist, the DPPIV inhibitor, the ACC inhibitor, the FXR agonist or the CCR2/5 dual antagonist. The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the PPAR agonist. The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the DPPIV inhibitor. The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the ACC inhibitor. The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the FXR agonist. The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the CCR 2/5 dual agonist.

The PPAR agonist may be elafibranor. The DPPIV inhibitor may be at least one of evogliptin and sitagliptin. The ACC inhibitor may be firsocostat. The FXR agonist may be obeticholic acid. The CCR 2/5 dual antagonist may be cenicriviroc.

The pharmaceutical composition may include the 3-(4-(3-(1-(5-ethylpyrimidin-2-yl)piperidin-4-yl)propoxy)-2,6-difluorophenyl)-5-isopropyl-1,2,4-oxadiazole and the elafibranor, evogliptin, sitagliptin, firsocostat, obeticholic acid or cenicriviroc.

The nonalcoholic fatty liver disease may be selected from the group consisting of nonalcoholic fatty liver, nonalcoholic steatohepatitis, liver fibrosis and cirrhosis.

The pharmaceutical composition may be a pharmaceutical composition for preventing or treating nonalcoholic fatty liver disease by inhibiting deposition of triglycerides in liver tissues.

The pharmaceutical composition may be a pharmaceutical composition for preventing or treating nonalcoholic fatty liver disease by inhibiting occurrence of inflammation in liver tissues.

The pharmaceutical composition may be a pharmaceutical composition for preventing or treating nonalcoholic fatty liver disease by inhibiting fibrosis of liver tissues.

When it is described in the present specification that the pharmaceutical composition includes A and B, the pharmaceutical composition may be construed to mean a composition including A and B; a combination including A and B, respectively, as a separate preparation; or a composition including the combination. Thus, in the present invention, the composition may be used interchangeably with the combination.

The pharmaceutical composition may be one which includes a compound represented by Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist, respectively, as a separate preparation or includes all in the form of a combination preparation.

The pharmaceutical composition may be a combination of separate preparations, or a combination preparation.

The pharmaceutical composition may include a first compartment containing a first active ingredient, and a second compartment containing a second active ingredient.

The first active ingredient may be a compound represented by Formula 1, pharmaceutically acceptable salts thereof, optical isomers thereof, hydrates thereof, solvates thereof or mixtures thereof. The second active ingredient may include at least one selected from a PPAR agonist, a DPPIV inhibitor, an ACC inhibitor, a FXR agonist and a CCR2/5 dual antagonist.

According to embodiments of the present invention, in the pharmaceutical composition, the first compartment containing the first active ingredient and the second compartment containing the second active ingredient may be co-administered, and thus the pharmaceutical composition may be a composition for co-administration. Specifically, the pharmaceutical composition may be a combination in which two ingredients are formulated into separate unit dosage forms. In this case, the first active ingredient and the second active ingredient may be administered in combination as separate dosage forms.

In one specific embodiment of the present invention, in a C57BL/6J mouse model in which nonalcoholic fatty liver disease was induced according to the supply of special feed, it was found that the pharmaceutical composition of one embodiment is effective in the treatment and prevention of nonalcoholic liver disease through histological examination, confirmation of concentration of inflammation and fibrosis-related proteins, blood AST and ALT measurement experiments, and the like.

More specifically, when administering the pharmaceutical composition according to one embodiment, it is confirmed that a nonalcoholic fatty liver disease (NAFLD) activity score is lowered, a relative proportion of lipid droplets per unit area is decreased, an infiltration of inflammatory cells in liver tissues is reduced, a fibrosis of liver tissues is inhibited, and an effect of ameliorating blood inflammatory and fibrotic biomarkers are increased, and a concentration of AST and ALT in blood is decreased, compared to when co-administering a compound represented by Formula 1, pharmaceutically acceptable salts thereof, optical isomers thereof, hydrates thereof, solvates thereof or mixtures thereof as a GPR119 agonist; or a PPAR agonist, a DPPIV inhibitor, an ACC inhibitor, a FXR agonist or a CCR2/5 dual antagonist, respectively.

In the present invention, a non-limiting example of the pharmaceutically acceptable salts of the compound represented by above formula 1 may include inorganic acid salts such as hydrochloric acid, bromic acid, phosphoric acid or sulfuric acid; organic carboxylic acid salts such as acetic acid, trifluoroacetic acid, citric acid, maleic acid, oxalic acid, succinic acid, benzoic acid, tartaric acid, fumaric acid, mandelic acid, ascorbic acid or malic acid, or sulfonic acid salts such as methanesulfonic acid or para-toluenesulfonic acid; alkali metal salts such as sodium, potassium or lithium; various acid salts known to be capable of forming other pharmaceutically acceptable salts; or the like.

The pharmaceutical composition for preventing or treating nonalcoholic fatty liver disease according to one embodiment may be used in the form of a general pharmaceutical preparation. The pharmaceutical preparation may be administered in several oral and parenteral dosage forms upon administration, and the dosage form may be variously determined according to a method of use.

If the pharmaceutical composition of one embodiment is formulated into several oral and parenteral dosage forms, it may be possible to use the generally used excipients such as fillers, diluents, extenders, binders, humectants, disintegrants, surfactants, etc.

A solid preparation for oral administration may include tablets, pills, powders, granules, capsules, etc., and these solid preparations may be prepared by mixing at least one excipient, for example, starch, calcium carbonate, sucrose, lactose, gelatin or the like in the pharmaceutical composition. In addition, lubricants such as magnesium stearate, talc, etc., may be used in addition to simple excipients.

Furthermore, a liquid preparation for oral administration may include suspending agents, liquids for internal use, emulsions, syrups, etc., but may also include several excipients, for example, humectants, sweetening agents, flavoring agents, preservatives, etc. in addition to water and liquid paraffin, which are the frequently used simple diluents.

A preparation for parenteral administration may include sterile aqueous solutions, non-aqueous solvents, suspensions, emulsions, lyophilized preparations and suppositories. The non-aqueous solvent and the suspending agent used herein may include propylene glycol, polyethylene glycol, vegetable oil like olive oil, injectable ester like ethyl oleate, etc. A base of the suppository used herein may include witepsol, macrogol, tween 61, cacao butter, laurinum, glycerogelatin, etc.

In addition, the pharmaceutical composition for preventing or treating nonalcoholic fatty liver disease according to the present invention may represent an effective amount in an administration range of about 1 to about 1,000 mg. An administered amount or an intake amount may be administered with a variety of administered doses and methods, such as once a day or several times a day by dividing the composition depending on a subject's body weight, age, gender, health condition, diet, administration time, administration method, excretion rate, and severity of the disease.

In the present invention, nonalcoholic fatty liver disease (NAFLD) may include both primary and secondary nonalcoholic fatty liver diseases. Specifically, in the present invention, nonalcoholic fatty liver disease (NAFLD) may include simple steatosis, nonalcoholic steatohepatitis (NASH), and liver fibrosis and liver cirrhosis caused by the progression of these diseases, but is not limited to.

The pharmaceutical composition of one embodiment may include a compound represented by Formula 1 or an active ingredient having a similar function thereto; and at least one selected from a PPAR agonist, a DPPIV inhibitor, an ACC inhibitor, a FXR agonist and a CCR2/5 dual antagonist.

The present invention may also provide a method for preventing or treating nonalcoholic fatty liver disease, including administering into a subject in need of treatment a therapeutically effective amount of a pharmaceutical composition including a compound represented by Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist.

In the present invention, the term "subject in need of treatment" may mean mammals including humans, and the term "administration" may refer to providing a predetermined material to subjects by any appropriate method. The term "therapeutically effective amount" may refer to an amount of an active ingredient or a pharmaceutical composition which induces animals or humans to show the biological or medical responses considered by investigators, veterinarians, doctors or other clinicians, and may include an amount thereof for inducing a relief of symptoms of diseases or disorders to be treated. It is apparent to those skilled in the art that a therapeutically effective dosage and the number of administration for active ingredient of the present invention may vary depending on a desired effect.

In one embodiment, the term, a route of administration of the pharmaceutical composition of the present invention may be administered through any general route as long as it can reach a target tissue.

Administration may be performed orally, intraperitoneally, intravenously, intramuscularly, subcutaneously, endothelially, intranasally, intrapulmonarily, rectally, intracavitarily, intraperitoneally and intrathecally, but is not limited thereto.

The pharmaceutical composition of one embodiment may be administered once a day or at least twice a day at a certain interval of time.

The pharmaceutical composition of one embodiment may be used alone or in combination with a surgery, endocrinotherapy, drug treatment and methods of using a biologic response modifier, in order to prevent and treat nonalcoholic fatty liver disease.

One embodiment may also provide a food composition for preventing or ameliorating nonalcoholic fatty liver disease, including a compound represented by above Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist as an active ingredient.

In one embodiment, the term "amelioration" may refer to all the acts, in which a disease gets better or takes a favorable turn by administering the composition.

In one embodiment, the term "food" may refer to meats, sausages, breads, chocolates, candies, snacks, confectioneries, pizzas, instant noodles, other noodles, chewing gums, dairy products including ice creams, various types of soup, beverages, teas, health drinks, alcohol beverages, vitamin complexes, health functional foods, health foods, health supplement foods and the like, and include all the foods in a conventional sense.

The term "health functional food" may be the same term as food for special health use (FoSHU), and refer to the food having a high medical and medicinal effect, which is processed to efficiently show a biomodulation function in addition to supplying nutrients. In this case, the "function(ality)" may refer to controlling nutrients or obtaining a beneficial effect on health uses such as a physiological action, etc., with regard to the structure and function of the human body.

The "health food" may refer to food having an effect of actively maintaining or enhancing health compared to general food, and the "health supplement food" may refer to food for a health supplement purpose. In some cases, the terms of health functional food, health food, and health supplement food may be used interchangeably.

The food of the present invention may be prepared by a method conventionally used in the art, and raw materials and ingredients conventionally added in the art may be added to prepare the food during the preparation. Specifically, proteins, carbohydrates, fats, nutrients, seasoning agents, and flavoring agents may be included, and an example of the carbohydrates may include glucose, fructose, maltose, sucrose, oligosaccharide, dextrin, cyclodextrin, xylitol, sorbitol, erythrol, saccharin or synthetic flavoring agents, but is not limited thereto. The food composition of the present invention may be prepared into various dosage forms without limitation, as long as the dosage form is recognized as food.

The present invention may also provide a method for preventing or ameliorating nonalcoholic fatty liver disease, including administering into a subject in need of amelioration a food composition including a compound represented by above Formula 1, pharmaceutically acceptable salts thereof, optical isomers thereof, hydrates thereof, solvates thereof, or mixtures thereof; and at least one selected from a PPAR agonist, a DPPIV inhibitor, an ACC inhibitor, a FXR agonist and a CCR2/5 dual antagonist as an active ingredient.

The present invention may also provide a feed composition for preventing or ameliorating nonalcoholic fatty liver disease, including a compound represented by above formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist as an active ingredient.

In the present invention, the term "feed" may refer to any natural or artificial diet, one meal, etc., or an ingredient of the one meal, which is to be consumed or digested by livestock or appropriate thereto. The feed may include feed additives or auxiliary feeds.

The kind of the feed is not particularly limited, and the feed conventionally used in the art may be used. A non-limiting example of the feed may include vegetable feeds such as grains, root fruits, food processing by-products, algae, fibers, pharmaceutical by-products, oils and fats, starches, oil meals, grain by-products or the like; animal feeds such as proteins, inorganic matters, oils and fats, minerals, single-cell proteins, zooplanktons, foods or the like. The feed may be used alone or by mixing at least two thereof.

The present invention may also provide a use of the pharmaceutical composition including a compound represented by above Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist as an active ingredient for preventing or treating nonalcoholic fatty liver disease.

The present invention may also provide a use of the pharmaceutical composition including a compound represented by above Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist as an active ingredient, in the manufacture of a medicament for preventing or treating nonalcoholic fatty liver disease.

The present invention may also provide a method for preventing or treating nonalcoholic steatohepatitis, including administering into a subject in need of treatment a therapeutically effective amount of a pharmaceutical composition including a compound represented by above Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist.

The present invention may also provide a combination of a compound represented by above Formula 1, pharmaceutically acceptable salts thereof, optical isomers thereof, hydrates thereof, solvates thereof or mixtures thereof; and at least one selected from a PPAR agonist, a DPPIV inhibitor, an ACC inhibitor, a FXR agonist and a CCR2/5 dual antagonist.

The present invention may also provide a pharmaceutical composition for preventing or treating nonalcoholic steatohepatitis, including a compound represented by above Formula 1, pharmaceutically acceptable salts thereof, optical isomers thereof, hydrates thereof, solvates thereof or mixtures thereof in combination with at least one selected from a PPAR agonist, a DPPIV inhibitor, an ACC inhibitor, a FXR agonist and a CCR2/5 dual antagonist.

In the therapeutic method, the food composition, the amelioration method, the feed composition, the use and the combination thereof, the compound represented by above Formula 1 may be specifically 3-(4-(3-(1-(5-ethylpyrimidin-2-yl)piperidin-4-yl)propoxy)-2,6-difluorophenyl)-5-isopropyl-1,2,4-oxadiazole.

Matters mentioned in each of the pharmaceutical composition, the therapeutic method, the food composition, the amelioration method, the feed composition, the use and combinations thereof according to an embodiment of the present invention are applied the same to each of embodiments, if not contradictory to each other.

### Advantageous Effects

A pharmaceutical composition according to the present invention shows an excellent effect of ameliorating lipid metabolism, reducing fat accumulation in liver tissues, and preventing histological damage caused by inflammation and fibrosis of liver tissues, and thus can be effectively used for preventing or treating nonalcoholic fatty liver disease.

### Brief Description of the Drawings

FIG. 1 shows an effect of co-administration on a change in concentration of total GLP-1 and active GLP-1 in postprandial plasma after a single oral administration of a drug to normal mice.
FIG. 2 shows an effect of administration in combination with a DPPIV inhibitor by comparing NAFLD activity scores before and after multiple-dose administration of a drug to mice with induced nonalcoholic steatohepatitis.
FIG. 3 shows an effect of administration in combination with a PPARα/δ agonist by comparing NAFLD activity scores before and after multiple-dose administration of a drug to mice with induced nonalcoholic steatohepatitis.
FIG. 4 shows an efficacy of administration in combination with a DPPIV inhibitor by quantifying a relative proportion of lipid droplets in liver tissues after multiple-dose administration of a drug to mice with induced nonalcoholic steatohepatitis.
FIG. 5 shows an efficacy of administration in combination with a PPARα/δ agonist by quantifying a relative proportion of lipid droplets in liver tissues after multiple-dose of a drug to mice with induced nonalcoholic steatohepatitis.
FIG. 6 shows an efficacy of administration in combination with a DPPIV inhibitor on a relative proportion of expression of galectin-3 as an indicator of activated macrophage in liver tissues after multiple-dose administration of a drug to mice with induced nonalcoholic steatohepatitis.
FIG. 7 shows an efficacy of administration in combination with a PPARα/δ agonist on a relative proportion of expression of galectin-3 as an indicator of activated macrophage in liver tissues after multiple-dose administration of a drug to mice with induced nonalcoholic steatohepatitis.
FIG. 8 shows an efficacy of administration in combination with a DPPIV inhibitor on a relative proportion of expression of a smooth muscle actin (aSMA) as an indicator of activated hepatic stellate cells in liver tissues after multiple-dose administration of a drug to mice with induced nonalcoholic steatohepatitis.
FIG. 9 shows an efficacy of administration in combination with a PPARα/δ agonist on a relative proportion of expression of α smooth muscle actin (aSMA) as an indicator of activated hepatic stellate cells in liver tissues after multiple-dose administration of a drug to mice with induced nonalcoholic steatohepatitis.
FIG. 10 shows an efficacy of administration in combination with a DPPIV inhibitor on an indicator of inflammation and fibrosis in plasma after multiple-dose administration of a drug to mice with induced nonalcoholic steatohepatitis.
FIG. 11 shows an efficacy of administration in combination with a DPPIV inhibitor on concentration of ALT and AST as an indicator of liver damage in plasma after multiple-dose administration of a drug to mice with induced nonalcoholic steatohepatitis.
FIG. 12 shows an effect of increasing an inhibitory ability of secretion of CCL2, TNFa and IL1β, which are inflammatory cytokines, by treatment in combination with above compound 1 and a PPARα/δ agonist, an ACC inhibitor, a FXR agonist or a CCR2/5 dual antagonist in a process of differentiating human monocytes into macrophage.
FIG. 13 shows an effect of increasing an ability to decrease an expression of fibrotic proteins by treatment in combination with above compound 1 and a PPARα/δ agonist, an ACC inhibitor or a FXR agonist in a process of activating primary human hepatic stellate cells with TGFβ.

### Mode for Invention

The features and advantages of the present invention as well as methods for achieving them will be apparent with reference to exemplary embodiments described in detail hereinafter. However, the present invention is not limited to the exemplary embodiments disclosed hereinafter, but will be implemented in various different forms. Hereinafter, the following exemplary embodiments will be suggested for better understanding of the present invention and are provided only for the purpose of completely illustrating the scope of the present invention to those skilled in the art, and thus the present invention will be defined only by the scope of the claims thereto.

Nonalcoholic steatohepatitis is not a disease caused by a single factor, and thus it may be difficult to expect an excellent therapeutic effect with a single drug. Accordingly, the pharmaceutical composition according to one embodiment of the present invention, including a plurality of active ingredients, may improve an effect of preventing or treating nonalcoholic steatohepatitis according to administration in combination with complementary drugs of various mechanisms.

Drugs, which are under current development as therapeutic agents for nonalcoholic steatohepatitis, are roughly classified into i) drugs for reducing fat accumulation in liver tissues through metabolic improvement, ii) drugs for reducing oxidative stress in liver tissues or inhibiting inflammatory responses through direct control thereof, iii) drugs for inhibiting apoptosis of hepatocytes, and iv) drugs for inhibiting accumulation of extracellular fibrotic proteins.

In particular, metabolic diseases such as obesity and diabetes have a high morbidity rate and have etiological characteristics in which a metabolic imbalance causes fat accumulation in liver tissues, and thus diabetic drugs with various mechanisms previously developed for the purpose of improving metabolism have been confirmed to be effective in animal models with nonalcoholic steatohepatitis or have been in clinical exploratory study.

The pharmaceutical composition according to one embodiment of the present invention may include a GPR119 agonist and at least one selected from a peroxisome proliferator-activated receptor (PPAR) agonist, a dipeptidyl peptidase IV (DPPIV) inhibitor, an acetyl coenzyme A carboxylase (ACC) inhibitor, farnesoid X receptor (FXR) agonist and C-C chemokine receptor 2/5 (CCR2/5) dual antagonist. Thus, the pharmaceutical composition may exhibit an excellent effect of treating or preventing nonalcoholic steatohepatitis by complementary effects of the active ingredients described above. More specifically, the pharmaceutical composition of one embodiment may exhibit a synergistic effect on the treatment or prevention of nonalcoholic steatohepatitis compared to the case of individually administering each of the above-described active ingredients.

In the present specification, "at least one" may be construed to indicate all combinations derived from a plurality of configurations. For example, "at least one of A, B and C" may be construed to include "A, B or C", "two selected from A, B and C" and all combinations derived from "A, B and C."

GPR119 may be one of G protein-coupled receptors (GPCRs) to which Gs is coupled to activate signaling through cAMP. GPR119 has a high expression level in pancreatic beta cells and L cells of small intestines, is also present in the liver, and serves as a regulator of fatty acid biosynthesis in the liver. In addition, when GP119 is activated, it is possible to control all of fatty liver, inflammation, and fibrosis in nonalcoholic steatohepatitis by inhibiting the differentiation and activation of inflammatory cells and the activation of hepatic stellate cells.

Mechanistically, when GPR119 in small intestines is activated by fatty acid derivatives derived from ingested food, GPR119 may increase the secretion of glucagon-like peptide-i (GLP-1). Secreted GLP-1 is inactivated by an enzyme called dipeptidyl peptidase IV (DPPIV) within minutes in the blood.

GLP-1 may have a pharmacological effect on metabolic improvement, and GLP-1-like peptide may ameliorate fatty liver in patients with nonalcoholic steatohepatitis in the clinic.

Referring to the above-described mechanism, when administering a GPR119 agonist (e.g., the compound represented by Formula 1 described above) in combination with a DPPIV inhibitor developed as a hypoglycemic agent, the duration of biological activity of active GLP-1 in the blood may be prolonged. Accordingly, it may be possible to show an effect of the GPR119 agonist on treating or preventing nonalcoholic steatohepatitis and a pharmacological effect of GLP-1 of ameliorating the metabolism at the same time.

In other words, the pharmaceutical composition of one embodiment, including both the GPR119 agonist and the DPPIV inhibitor, may complement effects of GLP-1 on ameliorating metabolism in addition to medicinal effects of the GPR119 agonist per se on ameliorating nonalcoholic steatohepatitis. Thus, the pharmaceutical composition may be regarded to show a synergistic effect of treating and preventing nonalcoholic steatohepatitis.

The GPR119 agonist may be a GPR119 ligand, and more specifically may be a compound represented by Formula 1 described above.

The pharmaceutical composition according to one embodiment may include various combinations of active ingredients in addition to the combination of the GPR119 agonist and the DDPIV inhibitor. For example, the pharmaceutical composition according to one embodiment may include the above-described PPAR agonist or ACC inhibitor as ingredients, which increase effectiveness for treating or preventing nonalcoholic steatohepatitis by enhancing a metabolic improvement effect in combination with the GPR119 agonist, in addition to the DPPIV inhibitor which is an ingredient exhibiting the effect of improving metabolism together with the GPR119 agonist. Alternatively, the pharmaceutical composition may include a FXR agonist or a CCR 2/5 dual antagonist, which may further ameliorate inflammation and fibrosis in patients with nonalcoholic steatohepatitis in combination with the GPR119 agonist.

More specifically, the PPAR agonist may be at least one of pioglitazone, lobeglitazone, elafibranor, lanifibranor and saroglitazar; the DPPIV Inhibitor may be at least one of sitagliptin, vildagliptin, saxagliptin, linagliptin, gemigliptin, anagliptin, teneligliptin, alogliptin, trelagliptin, omarigliptin, evogliptin, gosogliptin and dutogliptin; and the ACC inhibitor may be firsocostat. The FXR agonist may be at least one of obeticholic acid, tropifexor and GW4064 (3-(2,6-dichlorophenyl)-4-(3'-carboxy-2-chlorostilben-4-yl)oxymethyl-5-isopropylisoxazole). The CCR 2/5 dual antagonist maybe cenicriviroc.

The pharmaceutical composition of one embodiment may include the GPR119 agonist; and at least one of the PPAR agonist, the DPPIV inhibitor, the ACC inhibitor, the FXR agonist and the CCR2/5 dual antagonist described above.

The pharmaceutical composition of one embodiment may include the compound represented by Formula 1 described above; and at least one of the PPAR agonist, the DPPIV inhibitor, the ACC inhibitor, the FXR agonist and the CCR2/5 dual antagonist.

The pharmaceutical composition of one embodiment may include 3-(4-(3-(1-(5-ethylpyrimidin-2-yl)piperidin-4-yl)propoxy)-2,6-difluorophenyl)-5-isopropyl-1,2,4-oxadiazole; and at least one of pioglitazone, lobeglitazone, elafibranor, lanifibranor, saroglitazar, sitagliptin, vildagliptin, saxagliptin, linagliptin, gemigliptin, anagliptin, teneligliptin, alogliptin, trelagliptin, omarigliptin, evogliptin, gosogliptin and dutogliptin, firsocostat, obeticholic acid, tropifexor, GW4064, and cenicriviroc.

The pharmaceutical composition of one embodiment may include a combination of ingredients. Thus, the administration of the pharmaceutical composition of one embodiment may exhibit an excellent effect of treating or preventing nonalcoholic steatohepatitis compared to the case in which individual ingredients are administered alone.

Hereinafter, the pharmaceutical composition according to one embodiment of the present invention will be described in detail with reference to Examples and Comparative Examples.

### < Example 1> Confirmation of combination efficacy of GPR119 ligand and drugs of other mechanisms in mouse model with induced steatohepatitis due to supply of special feed

In order to confirm an effect of the pharmaceutical composition according to one embodiment of the present invention on treating nonalcoholic fatty liver disease, a following experiment was performed.

### Confirmation of mechanism of action in normal mice

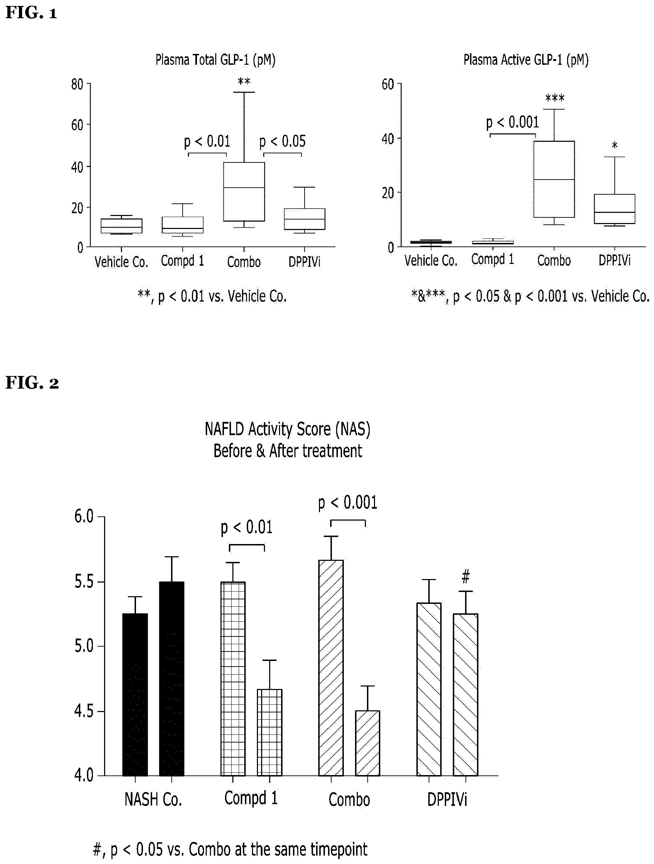

Seven-weeks-old male ICR mice were orally administered once with 3-(4-(3-(1-(5-ethylpyrimidin-2-yl)piperidin-4-yl)propoxy)-2,6-difluorophenyl)-5-isopropyl-1,2,4-oxadiazole (hereinafter, referred to as "compound 1") in the compound represented by Formula 1 and sitagliptin as a DPPIV inhibitor at a dose of 3 mg/kg and 10 mg/kg, respectively, alone or in combination. Glucose solution was orally loaded 30 minutes after administration. After oral glucose loading, blood was collected at a time point of 10 minutes and a total concentration of GLP-1 (ALPCO, 43-GPTHU-E01) and a concentration of active GLP-1 in plasma (ALPCO, 43-GP1HU-E01) were quantified by using an ELISA kit and the results thereof are shown in FIG. 1.

As confirmed in FIG. 1, when the GPR119 agonist and the DPPIV inhibitor were used in combination, the total concentration of GLP-1 and the peak concentration of active form in plasma were increased compared to when compound 1 was administered alone. This means that when drugs of both mechanisms are used in combination, a GLP-1 secretion in small intestines is increased and a biological half-life of GLP-1 in the blood is prolonged.

### Preparation of mouse model with nonalcoholic steatohepatitis

A six-weeks-old male C57BL/6J mouse was fed with a special feed of high fat, high fructose, and high cholesterol (Research Diet, D09100301) for at least 27 weeks, and thus was induced to develop nonalcoholic steatohepatitis. After that, compound 1 was administered alone or in combination so that a daily dose reached 100 mg/kg/day. As the DPPIV inhibitor, evogliptin at 200 mg/kg/day or sitagliptin at 150 mg/kg/day was administered alone or in combination. As the PPAR agonist, elafibranor was administered alone or in combination so as to reach 30 mg/kg/day. As a method of administration alone or in combination, the above-described compound 1, DPPIV inhibitor, and/or PPAR agonist were prepared as a drug-diet admixture and supplied for 8 to 12 weeks.

### Histological examination

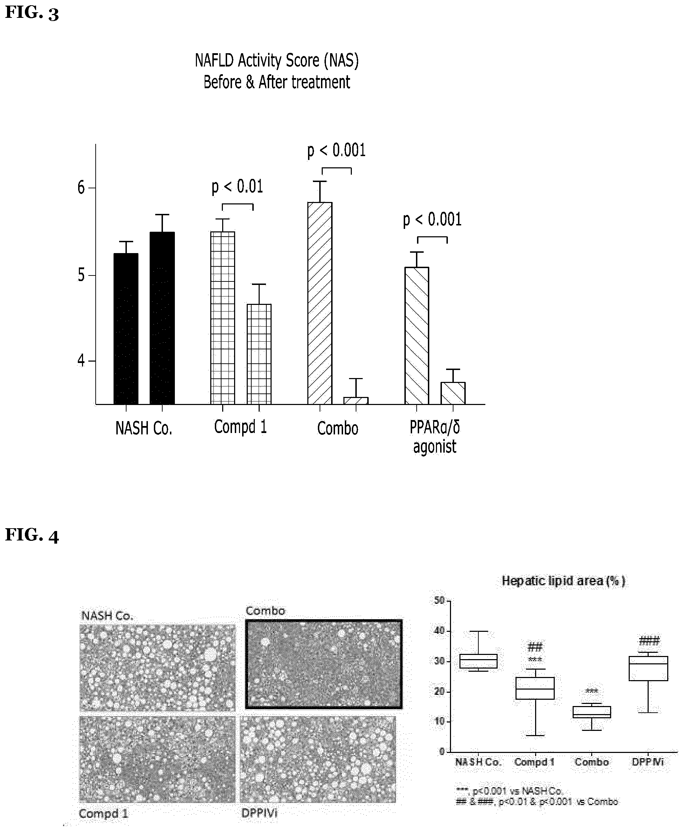

For a histological examination, liver tissues were isolated through biopsy and autopsy from the mouse model with induced nonalcoholic steatohepatitis prepared above before and after drug administration and were fixed in 10% formalin so as to prepare a paraffin block and obtain a 2 µm thick tissue section. After that, hematoxylin and eosin (HE) stain was performed by using an automatic stainer (Autostainer XL, Leica) in order to confirm an infiltration of inflammatory cells. A ratio of lipid droplets deposited in the stained liver tissues, a degree of infiltration of inflammatory cells and a degree of cell necrosis were expressed as NAFLD activity scores (NAS), and the results thereof are shown in FIG. 2 and 3.

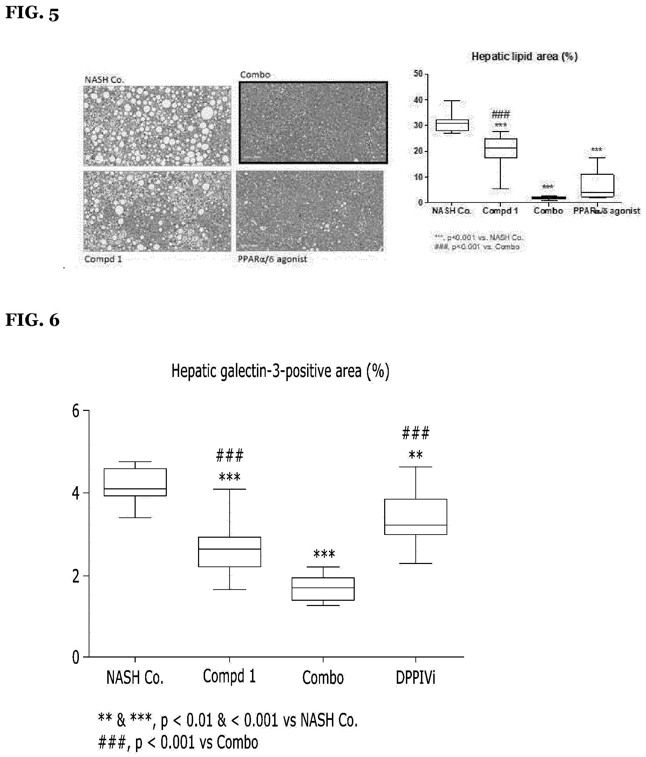

For quantitative comparison of fat accumulation in liver tissues, the HE-stained tissues were subjected to an image analysis to calculate a relative proportion of lipid droplets per unit area, thereby evaluating a medicinal efficacy of co-administration. The results thereof are shown in FIG. 4 and 5.

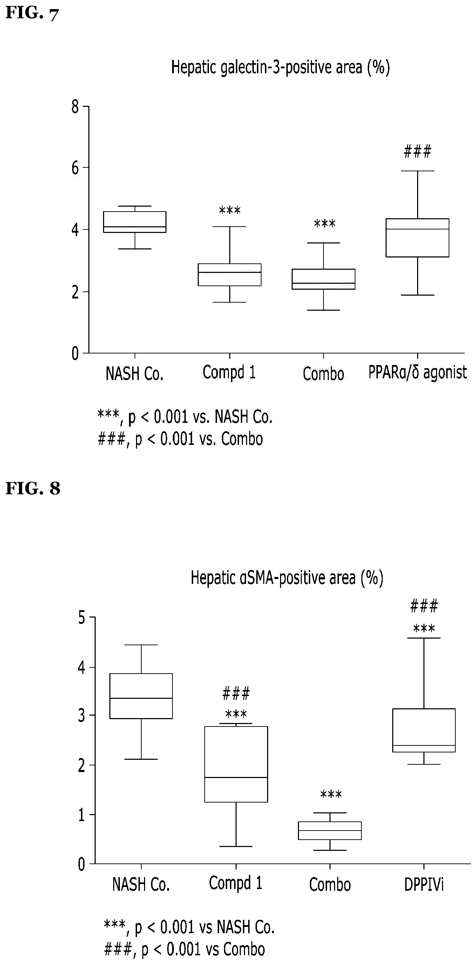

To compare the degree of infiltration of inflammatory cells in liver tissues, an expression level of galectin-3, an indicator of increased expression in activated macrophages, was measured through immunostaining. After that, a ratio of the area stained by the galectin-3 antibody in the unit area was calculated through image analysis so as to evaluate the medicinal efficacy of ameliorating inflammation according to the co-administration. The results thereof are shown in FIG. 6 and 7.

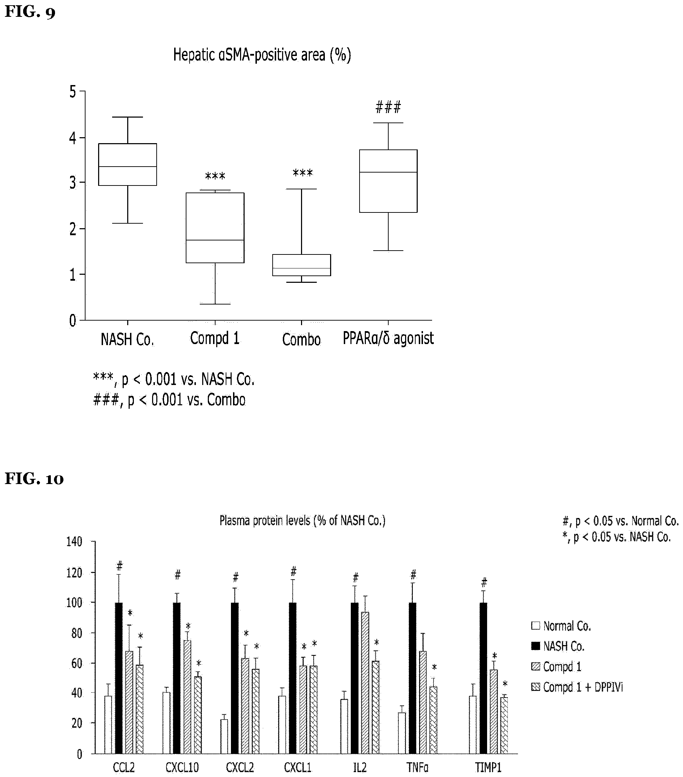

To evaluate the effectiveness on fibrosis, an expression of α smooth muscle actin (aSMA) in a liver tissue section was measured through immunostaining so as to compare the ratio of activated stellate cells. After that, a ratio of the area stained by the aSMA antibody in the unit area was calculated through image analysis. The results thereof are shown in FIG. 8 and 9.

### Confirmation of concentration of inflammation and fibrosis-related proteins

To evaluate an effect on concentration of inflammation (CCL2, CXCl10, CXCl2, IL2 and TNFa) and fibrosis-related protein (TIMP1) in plasma, a concentration of CCL2, CXCl10, CXCl2,IL2, TNFa (MSD, K15255D-1) and TIMP1 (R&D Systems, MTM100) proteins in the plasma obtained from the mouse model was quantified by using a commercially available ELISA kit. The results thereof are shown in Fig. 10.

### Measurement of blood AST and ALT

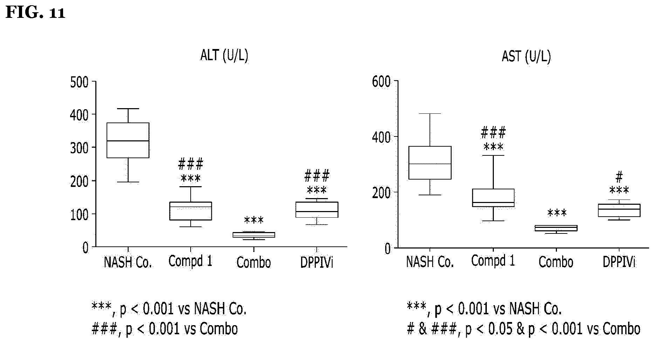

The mouse model with induced nonalcoholic steatohepatitis was subjected to autopsy, after which plasma was isolated therefrom, so as to quantify aspartate aminotransferase (AST) and alanine aminotransferase (ALT) by using an automatic blood analyzer (Konelab 20i). The results thereof are shown in Fig. 11.

As confirmed in FIG. 2, the NAS was significantly improved in mice administered with compound 1 alone compared to before treatment, but the effect of administering evogliptin alone, the DPPIV inhibitor, was insignificant. However, when both drugs were administered in combination, the medicinal efficacy of NAS improvement was remarkably increased.

Referring to FIG. 3, when compound 1 or elafibranor (a PPAR agonist), was administered alone, the NAS was significantly improved compared to before treatment, and when both drugs were administered in combination, the medicinal efficacy of NAS improvement was further increased.

Referring to FIG. 4, a size of lipid droplets in liver tissues was decreased in mice administered with compound 1 alone, and thus a relative area of lipid droplets was significantly reduced. When evogliptin, the DPPIV inhibitor, was administered alone, the relative area of lipid droplets showed a tendency of decreasing. Above all, when compound 1 was administered in combination with the DPPIV inhibitor, the relative area of lipid droplets in liver tissues was further reduced, thus confirming an increase in the medicinal efficacy of improving the fatty liver according to the combination of both drugs.

As confirmed in FIG. 5, as a result of quantitatively evaluating a degree of fat deposition in liver tissues, a ratio of fat in liver tissues was significantly reduced by the administration of compound 1 or the PPAR agonist alone. As a result of administering compound 1 in combination with the PPAR agonist, a ratio of lipid droplets area was further reduced, thus showing an improvement of 94% compared to a control group (NASH Co.). This indicates an increase in the medicinal efficacy of improving the fatty liver according to the combination of both drugs.

Referring to FIG. 6, it was shown that an expression of galectin-3, an indicator of activated macrophages in liver tissues, is significantly reduced in mice administered with compound 1 or evogliptin, the DPPIV inhibitor, alone, and when both drugs are used in combination, an effect of decreasing an expression of galectin-3 is further increased. This suggests that when both drugs are administered in combination, an effect of reducing an infiltration of inflammatory cells in liver tissues will be increased.

Referring to FIG. 7, an expression of galectin-3, an indicator of activated macrophages in liver tissues was significantly decreased in mice administered with compound 1 alone. When elafibranor, a PPAR agonist, was administered alone, there was no effect of significantly decreasing an expression of galectin-3, but when both drugs were used in combination, it was confirmed that an effect of reducing an expression of galectin-3 is further increased. This suggests an increase in the effect of reducing an infiltration of inflammatory cells in liver tissues according to the combination of both drugs.

Referring to FIG. 8, an expression of aSMA, which is an indicator of hepatic stellate cell activation inducing fibrosis in liver tissues, was significantly reduced in mice administered with compound 1 or evogliptin, the DPPIV inhibitor, alone. It was suggested that an effect of inhibiting an expression of aSMA is further increased when both drugs are administered in combination. This suggests that an effect of inhibiting fibrosis of liver tissues according to the combination of both drugs is increased.

Referring to FIG. 9, an expression of aSMA, an activation indicator of hepatic stellate cells inducing fibrosis in liver tissues, was significantly reduced in mice administered with compound 1 alone, while there was no effect of significantly reducing an expression of aSMA when elafibranor, the PPAR agonist, was administered alone. However, it was confirmed that an effect of inhibiting an expression of aSMA is further increased when both drugs are administered in combination. This suggests an increase in an effect of inhibiting fibrosis in liver tissues according to the co-administration of both drugs.

FIG. 10 shows that there is an effect on a concentration of blood inflammatory and fibrotic biomarkers when compound 1 is administered alone or in combination with sitagliptin, a DPPIV inhibitor. Plasma concentrations of CCL2, CXCl10 and CXCl2, chemokines that induce an infiltration of inflammatory cells, was increased in NASH mice, and those plasma concentrations thereof were significantly decreased when compound 1 was administered alone. When compound 1 was used in combination with the DPPIV inhibitor, the medicinal efficacy of reducing chemokines was further increased. Inflammatory cytokines, IL2 and TNFa, showed a tendency of decreasing by compound 1 alone, but was significantly decreased when combined with the DPPIV inhibitor. TIMP1, a fibrosis indicator, was also significantly increased in NASH mice, but was significantly decreased by administration of compound 1 alone, and a reduction effect was increased in combination with the DPPIV inhibitor. Accordingly, it is confirmed that an effect of improving plasma inflammatory and fibrotic biomarkers supporting histological changes is increased when compound 1 and the DPPIV inhibitor are used in combination.

FIG. 11 shows an effect of the pharmaceutical composition according to one embodiment on plasma ALT and AST as an indicator of liver damage. When compound 1 or evogliptin, a DPPIV inhibitor, was repeatedly administered alone, plasma concentrations of ALT and AST were significantly reduced. When compound 1 was administered in combination with the DPPIV inhibitor, ALT and AST concentrations were further decreased with a significant difference compared to administration of both drugs alone. This suggests that an effect of preventing damage to hepatocytes caused by nonalcoholic steatohepatitis is increased when compound 1 and the DPPIV inhibitor are administered in combination.

The above results show that administration of the pharmaceutical composition according to one embodiment of the present invention in combination with the GPR119 agonist and an agent for ameliorating metabolic diseases such as the DPPIV inhibitor or the PPAR agonist ameliorates fatty liver and inhibits liver damage, inflammation and fibrosis, thereby providing an effect on nonalcoholic fatty liver disease and exhibiting a more effective therapeutic effect.

### < Example 2> Confirmation of combination efficacy of GPR119 ligand and drugs of other mechanisms in human macrophages

### Confirmation of concentration of inflammatory cytokine proteins secreted in medium

In order to evaluate in vitro the possibility of increasing an effect of ameliorating inflammation among effects of ameliorating nonalcoholic fatty liver disease according to treatment with the GPR119 ligand in combination with drugs of other mechanisms, inflammatory cytokines in a medium were quantified to confirm an effect of combination of the GPR119 ligand and drugs of other mechanisms on the differentiation of human monocytes.

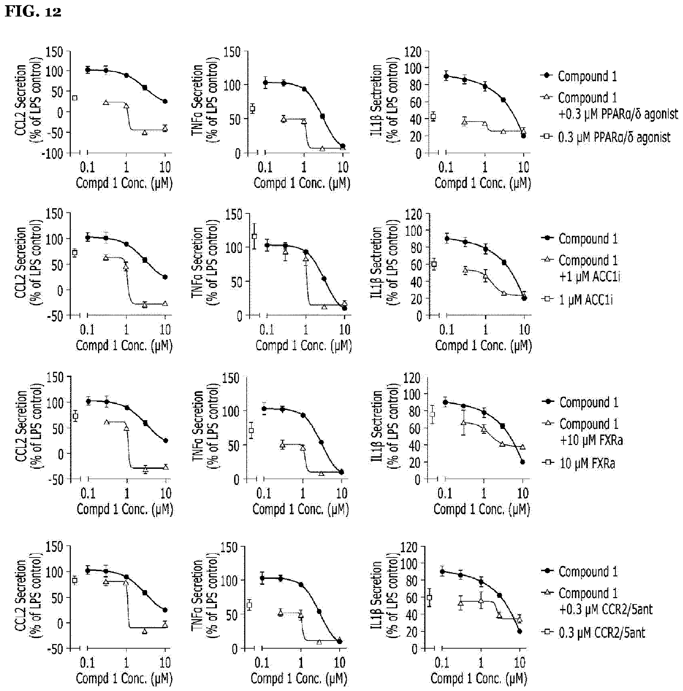

A human monocyte cell line (THP-1, ATCC^{®} TIB-202^{™}) was treated with 50 ng/ml of phorbol 12-myristate 13-acetate (PMA) for 48 hours and differentiated into macrophages, while being treated with compound 1 in combination with elafibranor as a PPAR agonist, firsocostat as an ACC inhibitor, obeticholic acid as a FXR agonist or cenicriviroc as a CCR2/5 dual antagonist, then replaced with a serum-free medium, and then treated with lipopolysaccharides (LPS; 0.5 ng/ml) for four hours, after which interleukin-1β (IL-1β; R&D Systems, DY201), tumor necrosis factor α (TNFa; R&D Systems, DY210), and C-C motif chemokine ligand 2 (CCL2; R&D Systems, DY279), which are inflammatory cytokines secreted into the medium, were quantified with a commercially available ELISKA kit, so as to evaluate an effect on monocyte differentiation, and the results thereof are shown in FIG. 12.

As confirmed in FIG. 12, when treated with compound 1 of the present invention during the process of differentiation of monocytes into macrophages, it was confirmed that IL-1β, TNFa and CCL2 secretion according to macrophage activation was decreased in a concentration-dependent manner. When treated with the PPAR agonist, the ACC inhibitor, the FXR agonist or the CCR2/5 dual antagonist in combination, an inhibitory ability of compound 1 on inflammatory cytokine secretion was increased.

The above results indicate that compound 1 of the present invention inhibits differentiation of immune cells and suppresses secretion of inflammatory cytokines caused by innate immunity, thereby inhibiting the progression of nonalcoholic fatty liver disease, and thus this action may be increased when treated in combination with the PPAR agonist, the ACC inhibitor, the FXR agonist or the CCR2/5 dual antagonist.

### < Example 3> Confirmation of combination efficacy of GPR119 ligand and drugs of other mechanisms in human hepatic stellate cells

### Confirmation of expression level of fibrotic protein expressed in cells

In order to evaluate in vitro the possibility of increasing an effect of inhibiting fibrosis among effects of ameliorating nonalcoholic fatty liver disease according to co-treatment of the GPR119 ligand and drugs of other mechanisms, a concentration of fibrotic proteins expressed in primary human hepatic stellate cells was quantified.

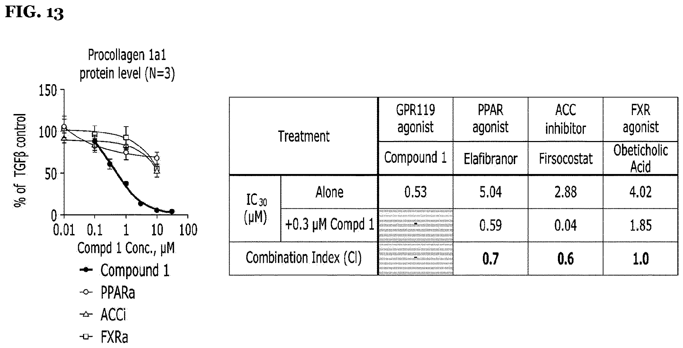

Primary human hepatic stellate cells (ScienCell, #5300) were stabilized for 24 hours, then cultured in a serum-free medium for 24 hours, and then treated with compound 1 in combination with elafibranor as a PPAR agonist, firsocostat as an ACC inhibitor, or obeticholic acid as a FXR agonist together with 5 ng/ml of recombinant tumor growth factor β1 (TGFβ1) after which the resulting cells were harvested in 18 hours later and an amount of pro-collagen 1a1 in total cell proteins was quantified with a commercialized ELISKA Kit (R&D Systems, DY6220), while an amount of total cell proteins was quantified and corrected by a BCA method, and the results of calculating a concentration response curve of each drug and a concentration (IC₃₀) showing 30% inhibitory activity are shown in FIG. 13.

As can be confirmed from FIG. 13, when compound 1 was treated alone, a protein expression of pro-collagen 1a1 induced by TGFβ 1 was inhibited in a concentration-dependent manner, and a 30% inhibitory concentration was calculated to be 0.55 µM. The PPAR agonist, the ACC inhibitor or the FXR agonist showed a relatively low response. The 0.3 µM of above compound 1 was treated along with the PPAR agonist, the ACC inhibitor or the FXR agonist at a concentration expected to induce 30% inhibition upon co-treatment, so as to calculate a concentration at which an expression of procollagen 1a1 is inhibited 30%, and calculate a fraction of concentration at which both drugs exhibit 30% inhibitory activity when co-treated compared to when treated with each drug alone, so that a sum thereof is expressed as a combination index (CI) (Chou TC & Talalay P., Adv Enzyme Regul, 1984:22:27-55). When compound 1 was treated in combination with the PPAR agonist or the ACC inhibitor, the CI was calculated to be 0.7 and 0.6, which was smaller than 1, thus confirming a synergistic effect. And the CI was calculated to be 1 when treated in combination with the FXR agonist, thus confirming an additive effect.

The above results indicate that compound 1 of the present invention inhibits activation of hepatic stellate cells to suppress expression of collagen, which is a fibrosis protein, thereby inhibiting the progression of nonalcoholic fatty liver disease, and thus this action may be increased when treated in combination with the PPAR agonist, the ACC inhibitor or the FXR agonist.

## Claims

1. A pharmaceutical composition for preventing or treating nonalcoholic steatohepatitis, comprising:
a compound represented by a following chemical Formula 1, pharmaceutically acceptable salts thereof, optical isomers thereof, hydrates thereof, solvates thereof or mixtures thereof; and
at least one selected from a PPAR agonist, a DPPIV inhibitor, an ACC inhibitor, a FXR agonist and a CCR2/5 dual antagonist: 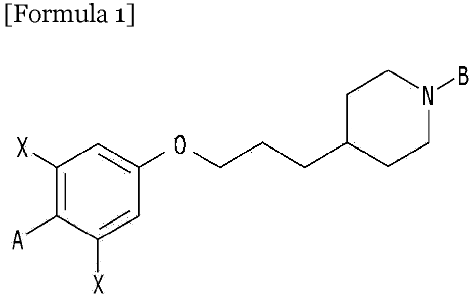 wherein in above Formula 1,
A is an oxadiazole group, a dihydrooxazole group, a thiazole group or a thiadiazole group, in which above A is unsubstituted or substituted with at least one substituent selected from the group consisting of a halogen atom, a C1-C6 straight or branched chain alkyl group, and a C1-C6 straight or branched chain hydroxyalkyl group, in which the alkyl group or the hydroxyalkyl group is each independently unsubstituted or substituted with a halogen atom or a C1-C6 alkoxy group;
B is a pyridine group, a pyrimidine group, a pyrazine group or an oxadiazole group, in which above B is unsubstituted or substituted with at least one substituent selected from the group consisting of a halogen atom, a C1-C6 straight or branched chain alkyl group, a C1-C6 straight or branched chain hydroxyalkyl group, a C1-C6 alkoxy group and an oxadiazole group, in which the C1-C6 straight or branched chain alkyl group, the C1-C6 straight or branched chain hydroxyalkyl group, the C1-C6 alkoxy group or the oxadiazole group is each independently unsubstituted or substituted with halogen, a C1-C6 alkyl group or a C1-C6 alkoxy group; and
X is F, Cl, Br or I.

2. The pharmaceutical composition for preventing or treating nonalcoholic fatty liver disease according to claim 1, wherein above A is 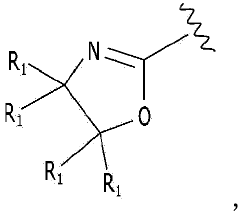 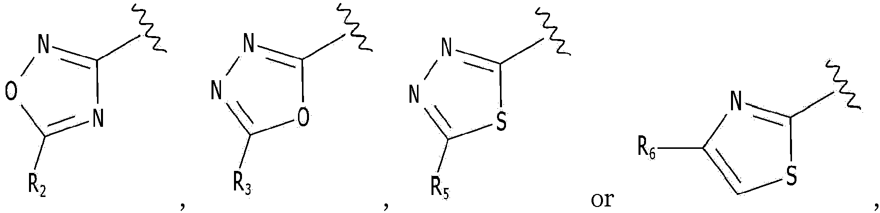 and
R₁ to R₃, R₅ and R₆ are each independently at least one substituent selected from the group consisting of a hydrogen atom, a halogen atom, a C1-C6 straight or branched chain alkyl group and a C1-C6 straight or branched chain hydroxyalkyl group, in which the alkyl group or the hydroxyalkyl group is each independently unsubstituted or substituted with a halogen atom or a C1-C6 alkoxy group.

3. The pharmaceutical composition for preventing or treating nonalcoholic fatty liver disease according toclaim 1, wherein above B is 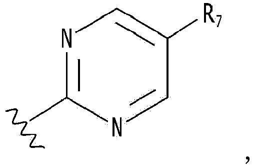 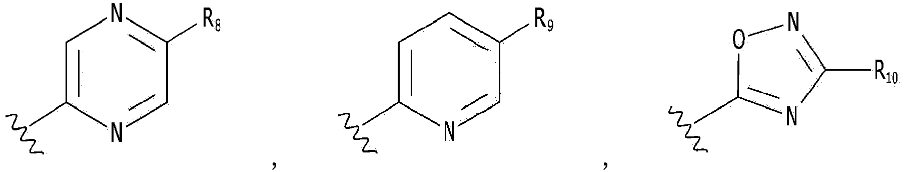 or 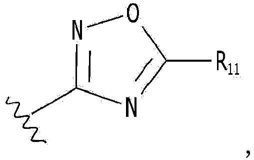 and
R₇ to R₁₁ are each independently substituted with at least one substituent selected from the group consisting of a hydrogen atom, a halogen atom, a C1-C6 straight or branched chain alkyl group, a C1-C6 straight or branched chain hydroxyalkyl group, a C1-C6 alkoxy group and an oxadiazole group, in which the alkyl group, the hydroxyalkyl group, the alkoxy group or the oxadiazole group is each independently unsubstituted or substituted with a halogen atom, a C1-C6 alkyl group or a C1-C6 alkoxy group.

4. The pharmaceutical composition for preventing or treating nonalcoholic fatty liver disease according to claim 1, wherein X is F.

5. The pharmaceutical composition for preventing or treating nonalcoholic fatty liver disease according to claim 1, wherein above A is an oxadiazole group substituted with a C1-C6 straight or branched chain alkyl group, above B is a pyrimidine group substituted with a C1-C6 straight or branched chain alkyl group, and X is F.

6. The pharmaceutical composition for preventing or treating nonalcoholic fatty liver disease according to claim 1, wherein the compound represented by above formula 1 is 3-(4-(3-(1-(5-ethylpyrimidin-2-yl)piperidin-4-yl)propoxy)-2,6-difluorophenyl)-5-isopropyl-i,2,4 -oxadiazole.

7. The pharmaceutical composition for preventing or treating nonalcoholic fatty liver disease according to claim 6, wherein the pharmaceutical composition comprises the PPAR agonist, the DPPIV inhibitor, the ACC inhibitor, the FXR agonist and the CCR2/5 antagonist.

8. The pharmaceutical composition for preventing or treating nonalcoholic fatty liver disease according to claim 6, wherein the pharmaceutical composition comprises at least one of elafibranor, evogliptin, sitagliptin, firsocostat, obeticholic acid and cenicriviroc.

9. The pharmaceutical composition for preventing or treating nonalcoholic fatty liver disease according to claim 7, wherein the PPAR agonist is elafibranor.

10. The pharmaceutical composition for preventing or treating nonalcoholic fatty liver disease according to claim 7, wherein the DPPIV inhibitor is at least one of evogliptin and sitagliptin.

11. The pharmaceutical composition for preventing or treating nonalcoholic fatty liver disease according to claim 7, wherein the ACC inhibitor is firsocostat.

12. The pharmaceutical composition for preventing or treating nonalcoholic fatty liver disease according to claim 7, wherein the FXR agonist is obeticholic acid.

13. The pharmaceutical composition for preventing or treating nonalcoholic fatty liver disease according to claim 7, wherein the CCR2/5 antagonist is cenicriviroc.

14. The pharmaceutical composition for preventing or treating nonalcoholic fatty liver disease according to claim 1, wherein the nonalcoholic fatty liver disease is selected from the group consisting of simple fatty liver, nonalcoholic steatohepatitis, liver fibrosis and cirrhosis.

15. The pharmaceutical composition for preventing or treating nonalcoholic fatty liver disease according to claim 1, wherein the pharmaceutical composition inhibits deposition of triglycerides in liver tissues.

16. The pharmaceutical composition for preventing or treating nonalcoholic fatty liver disease according to claim 1, wherein the pharmaceutical composition inhibits occurrence of inflammation in liver tissues.

17. The pharmaceutical composition for preventing or treating nonalcoholic fatty liver disease according to claim 1, wherein the pharmaceutical composition inhibits fibrosis of liver tissues.

18. Use of a pharmaceutical composition, comprising:
the compound represented by the chemical Formula 1 according to claim 1, pharmaceutically acceptable salts thereof, optical isomers thereof, hydrates thereof, solvates thereof, or mixtures thereof; and
at least one selected from a PPAR agonist, a DPPIV inhibitor, an ACC inhibitor, a FXR agonist and a CCR2/5 dual antagonist as an active ingredient for preventing or treating nonalcoholic fatty liver disease.

19. Use of a pharmaceutical composition, comprising:
the compound represented by the chemical Formula 1 according to claim 1, pharmaceutically acceptable salts thereof, optical isomers thereof, hydrates thereof, solvates thereof, or mixtures thereof; and
at least one selected from a PPAR agonist, a DPPIV inhibitor, an ACC inhibitor, a FXR agonist and a CCR2/5 dual antagonist as an active ingredient in the manufacture of a medicament for preventing or treating nonalcoholic fatty liver disease.

20. A method for preventing or treating nonalcoholic fatty liver disease, the method comprising: administering a therapeutically effective amount of a pharmaceutical composition including the compound represented by the chemical Formula 1 according to claim 1, pharmaceutically acceptable salts thereof, optical isomers thereof, hydrates thereof, solvates thereof, or mixtures thereof; and at least one selected from a PPAR agonist, a DPPIV inhibitor, an ACC inhibitor, a FXR agonist and a CCR2/5 dual antagonist as an active ingredient, into a subject in need of treatment.
